# EUROPEAN PATENT SPECIFICATION

(11) **EP 3 645 498 B1**
(45) Date of publication and mention of the grant of the patent: **04.08.2021**
(21) Application number: 18732118.7
(22) Date of filing: 26.06.2018
(51) Int. Cl.: C07C 51/48, C07C 53/08

(54) **PROCESS FOR RECOVERING ACETIC ACID FROM AN AQUEOUS STREAM COMPRISING THE SAME**
VERFAHREN ZUR RÜCKGEWINNUNG VON ESSIGSÄURE AUS EINEM WÄSSRIGEN STROM DAMIT
PROCÉDÉ DE RÉCUPÉRATION D'ACIDE ACÉTIQUE PROVENANT DE FLUX AQUEUX EN CONTENANT

(30) Priority: 29.06.2017 EP 17178753
(43) Date of publication of application: 06.05.2020
(73) Proprietor: Nouryon Chemicals International B.V., 6824 BM Arnhem (NL)
(72) Inventor: GÓRAK, Andrej, 58453 Witten (DE); SKIBOROWSKI, Mirko, 47058 Duisburg (DE); KUHLMANN, Hanns, 44549 Sprockhövel (DE); RANFT, Daniel, 45711 Datteln (DE)
(74) Representative: LKGlobal UK Ltd.
(86) International application number: PCT/EP2018/067020
(87) International publication number: WO 2019/002240

(56) References cited:
- CN-A- 101 172 940
- US-A- 4 353 784

## Description

The present invention relates to a process for recovering acetic acid from an aqueous stream comprising the same. Said aqueous stream optionally further comprises furfural and/or methanol.

Acetic acid is one of the world's most important chemicals. The global demand for acetic acid is about 6.5 million metric tons per year (Mt/a). In addition to use as household vinegar, acetic acid is mainly used in the production of polyvinyl acetate, cellulose acetate, and in the production of purified terephthalic acid, which is used to produce polyethylene terephthalate.
Acetic acid is produced industrially both synthetically and by bacterial fermentation. Acetic acid for use in the chemical industry is still predominantly made by the carbonylation of methanol. Other production routes include liquid-phase oxidation of saturated hydrocarbons or oxidation of ethylene or ethane. Yet another route is the fermentation route wherein ethanol, sugar or biomass is fermented, in the course of which acetic acid is formed.
In some of the production routes and also in most recycle streams, the acetic acid needs to be recovered from a diluted aqueous solution. For example in the production of acetic acid by pyrolysis of biomass, the acetic acid needs to be recovered from an aqueous stream comprising said acetic acid. Aqueous waste streams from pulp producers may also comprise acetic acid.
The challenge faced by industry is to recover the acetic acid from the aqueous streams in an economic manner.

In literature, several methods have been described for the recovery of acetic acids from aqueous solutions. A well-known route is via precipitation. However, this has the disadvantage that large amounts of gypsum or other salts are formed. Another route is by allowing the acetic acid to react with an alcohol, after which the formed acetic acid ester is recovered. Yet another known method is to use various membrane techniques to recover the acetic acid. Furthermore, many separation methods exist that are based on affinity. For all of these methods, a balance needs to be found between sufficiently good affinity to achieve a good separation and a sufficiently easy regeneration step. One of the affinity based recovery methods is liquid-liquid extraction, in which the acetic acid is recovered by transfer to an organic acid. Such a process comprises two parts. First, the acetic acid is extracted into the solvent. Subsequently, the solvent is regenerated so that substantially pure acetic acid is obtained. As extraction is often an environmentally friendly and mild method, it has been the subject of many research studies.
C.S. Lopez-Garzon and A.J.J. Straathof in Biotechnol. Adv. (2014), http://dx.doi.org/10.2016/j.biotechadv.2014.04.002, for example, provide a good overview of extraction methods that might be used for the recovery of carboxylic acids in general. They describe that in order to achieve an efficient extraction of these carboxylic acids from dilute aqueous solutions, extraction solvents are generally composed of
(i) an extractant,
(ii) a diluent,
and optionally (iii) a modifier The extractant (i) is the active component primarily responsible for the transfer of the carboxylic acid to the solvent phase by forming a complex with the carboxylic acid.
H.M. IJmker et al. in Separation and Purification Technology 125 (2014) 256-263 describe that there are three main groups of extractants considered in literature, which are
I. carbon-bonded oxygen bearing extractants such as alcohols or ethers
II. organophosphorous extractants such as trioctylphophine oxide (TOPO) and tributylphosphate (TBP)
III. aliphatic amine extractants such as tri-n-octylamine (TOA)

The diluent (ii) is responsible for dissolving the complex in the solvent phase. Typical diluents are alkanes, alcohols and halogenated hydrocarbons. If the diluent does not have the required solvation power, the complex will form a separate third phase, which will lead to separation problems. In that case, a modifier may be added which diminishes the separation problems by improving the solvation of the complex. Long chain alcohols are the most frequently used modifiers.

CN 101172940 discloses that an extraction agent which contains 6-24 C tertiary amine(s), n-alkyl alcohol and coal oil can be used for recovering acetic acid from dilute acetic acid or aldehyde wastewater. The third component, viz. coal oil, is added in order to improve the flow characteristics. Separation of this component from the extract phase might complicate solvent recovery.
CN 103772329 discloses a method for purifying a stream comprising acetic acid and furfural using an extraction solvent. A mixture of a tertiary amine containing 12-16C, an alcohol containing more than 7 carbon atoms, and nitro-containing aromatic hydrocarbon derivatives is used as the extraction solvent. Also in this case, separation of the nitro-containing aromatic hydrocarbon derivatives might complicate solvent recovery.
WO 200014052 discloses a microbial process for the preparation of acetic acid as well as solvents for its extraction from the fermentation broth. It is described that separation of the desired biologically produced product(s) from the permeate or centrifugate occurs by passing the permeate or centrifugate to an extractor where it is contacted with a solvent such as a di-alkyl and tri-alkyl amine in a suitable cosolvent, or tributyl phosphate, ethyl acetate, tri-octyl phosphine oxide and related compounds in a co-solvent. Suitable cosolvents are mentioned to be long chain alcohols, hexane, cyclohexane, chloroform, and tetrachloroethylene.
US 4353784 discloses a method of recovering acetic acid by extracting from an acetic acid-containing aqueous solution with an organic extracting agent and subjecting the extract to distillation, said method comprising the first step of performing extraction by using a tertiary amine having a boiling point higher than that of acetic acid and being capable of forming a non-aqueous phase as organic extracting agent in combination with an oxygen-containing, high-boiling point organic solvent selected from the group consisting of di-isobutylcarbinol, isophorene, methyl benzoate, tributyl phosphate, 3,3,5-trimethylcyclohexanone and 2-ethoxyethyl acetate, the second step of performing dehydration by subjecting the extract to distillation, and the third step of subjecting the dehydrated mixture to distillation in a reduced pressure distillation column at a column bottom temperature of 120 to 170°C to distill acetic acid and separate it from the organic extracting agent.
J.M. Wardell and C. Judson King in Journal of Chemical and Engineering Data, 23(2), 1978, 144-148 describe a study wherein phosphoryl and tertiary amine solvents and solvent mixtures have been investigated for extraction of acetic and formic acids from water.

The object of the present invention is to provide an improved process for recovering acetic acid from an aqueous stream comprising acetic acid.
It has surprisingly been found that acetic acid can be effectively recovered from an aqueous stream comprising acetic acid by subjecting said aqueous stream to a liquid-liquid extraction step wherein a specific combination of extractant and diluent is used.
More particularly, the present invention relates to a process for recovering acetic acid from an aqueous stream comprising acetic acid, said process comprising the steps of
(a) contacting the aqueous stream with tri-n-octylamine and 1-undecanol, with the weight ratio between tri-n-octylamine and 1-undecanol being between 0.8 : 1 and 5 : 1, to produce
   an organic phase (I) comprising acetic acid, tri-n-octylamine and 1-undecanol, and
   an aqueous phase (II);
   and
(b) isolating acetic acid from the organic phase.

Preferably, the weight ratio between tri-n-octylamine and 1-undecanol is between 1 : 1 and 3 : 1. The amount of tri-n-octylamine used in the process according to the invention is preferably the same as or greater than the amount of 1-undecanol used, based on weight. This is especially important if acetic acid is present in the aqueous stream in a relatively high amount (i.e. the aqueous stream comprises 5 wt% or more HAc, based on the total weight of the aqueous stream). The amount of tri-n-octylamine used is preferably the same as or greater than the amount of 1-undecanol used, based on weight, as the extraction efficiency decreases if the amount of tri-n-octylamine - acetic acid complex is higher than can be dissolved in the 1-undecanol. In a particularly preferred embodiment, the weight ratio between tri-n-octylamine and 1-undecanol is between 1.5 : 1.

The process according to the present invention is preferably used to recover acetic acid from an aqueous stream comprising at least 0.5% by weight of acetic acid, based on the total weight of the aqueous stream (which can also be denoted as an aqueous stream comprising an acetic acid mass fraction of 0.005). More preferably, the aqueous stream from which acetic acid is recovered via the method according to the present invention comprises at least 1% by weight of acetic acid, based on the total weight of the aqueous stream (mass fraction of 0.01) and even more preferably, it comprises at least 3% by weight of acetic acid, based on the total weight of the aqueous stream (mass fraction of 0.03). Preferably, the aqueous stream which is used in the process according to the present invention does not comprise more than 40% by weight of acetic acid, based on the total weight of the aqueous stream (which can also be denoted as an aqueous stream comprising an acetic acid mass fraction of 0.40). More preferably, said aqueous stream comprises at most 35% by weight of acetic acid, based on the total weight of the aqueous stream (mass fraction of 0.35), and most preferably, it comprises at most 30% by weight of acetic acid, based on the total weight of the aqueous stream (mass fraction of 0.30).

In one embodiment of the invention, an aqueous stream is used originating from a bio-based route towards acetic acid, such as fermentation of ethanol, sugar or biomass. As a result, said aqueous stream comprises besides acetic acid one or more compounds selected from the group consisting of methanol, furfural, formic acid, other organic acids, phenolics, furanics, sugar derivatives.

In step (a) of the process according to the present invention, the aqueous stream comprising acetic acid is contacted with tri-n-octylamine and with 1-undecanol (with the combined amounts of tri-n-octylamine and 1-undecanol being denoted as extractant mixture) in such a way that the weight ratio between tri-n-octylamine and 1-undecanol lies between 0.8 : 1 and 5 : 1.

Preferably, the weight ratio between the extractant mixture and the amount of acetic acid which is present in the aqueous stream is between 0.1 and 5, more preferably between 0.3 and 3, and most preferably between 0.5 and 1, as that way there is a good balance between extraction efficiency and the economics of solvent recovery. It has been found that the distribution coefficient tends to increase when the weight ratio between extractant mixture and acetic acid is increased. However, when it comes to regeneration of the solvent, the weight ratio between extractant mixture and acetic acid should be as low as possible.

Step (a) of the process according to the present invention is preferably conducted at a temperature of at least 15°C, and more preferably at a temperature of at least 20°C. Preferably, step (a) is conducted at a temperature of at most 60°C, more preferably of at most 45°C. The pressure at which step (a) is carried out is preferably at least 0.1 MPa, more preferably at least 0.5 MPa. The pressure during step (a) is preferably at most 5 MPa, more preferably at most 1.5 MPa. Most preferably, step (a) of the process according to the present invention is carried out at atmospheric pressure.

In step (b) according to the present invention, acetic acid is preferably isolated from the organic phase via one or more distillation steps.
Preferably, three distillation steps are performed in order to recover the solvent and obtain pure acetic acid (i.e. acetic acid which is at least 99% pure). After the extraction step, the organic phase comprises acetic acid, tri-n-octylamine, 1-undecanol, and some water. The organic phase may furthermore comprise furfural (depending on the source of the aqueous stream which was subjected to the extraction process according to the present invention). In a first distillation step, tri-n-octylamine and 1-undecanol are recovered from the organic phase (these components end up in the bottom product of the distillation). Preferably, the thus recovered tri-n-octylamine and 1-undecanol are recycled to the extraction step (step (a) of the process according to the present invention). Acetic acid, water and, optionally, furfural end up in the distillate of the first distillation step. If desired, a second distillation step is performed, wherein water is separated as distillate. The bottom product of said second distillation step comprises acetic acid and optionally furfural. Subsequently, if desired, a third distillation step is performed wherein pure acetic acid (i.e. acetic acid which is at least 99% pure) is obtained as distillate.
The distillation steps can be performed in any conventional distillation column, preferably at ambient pressure.

As an alternative to distillation the method according to L.J. Poole and C.J. King in Ind. Eng. Chem. Res. 1991, 30, 923-929 can be used. In that case, in step (b), acetic acid is isolated from the organic phase by back-extraction with trimethylamine, in which case a trimethylammonium salt is formed, which is subsequently thermally cracked in order to regenerate the trimethylamine. Yet other alternatives to distillation are the back-extraction methods as summarized by C.J. King et al. in Solvent Extraction, 1990, T. Sekine (Editor), pages 1791-1796, in which case in step (b) acetic acid is isolated from the organic phase by back-extraction following a temperature increase, back-extraction following a change in diluent composition, or back extraction using a pH-swing.

Although this is not preferred, it is feasible to use a modifier in the process according to the present invention to further improve the solvation of the formed extractant - acetic acid complex in the solvent phase. If a modifier is used, any modifier known in the art may be employed, but a long chain alcohol is preferred.

The acetic acid which is recovered via the process of the present invention can be used for any use known in the art. Typically it is used in the production of petrochemical intermediates or products, such as in the production of vinyl acetate monomer, terephthalic acid, acetate esters, cellulose acetate, acetic anhydride or monochloroacetic acid.

The aqueous phase which is retrieved in the process of the present invention can be discarded, preferably after being treated in a wastewater treatment plant to remove any remaining organic compounds.

The process according to the present invention is further illustrated by the following non-limiting examples.

### EXAMPLES

As extractants the following components were tested:
TOA (trioctylamine), TBP (tri-n-butyl phosphate), and TOPO (tri-octylphosphine oxide). As diluents, the following components were tested:
n-tetradecane, 2-undecanone, 1-undecanol, phenyldodecane.

**Table 1: List of extractants and diluents for the extraction of HAc**

| **Compound** | **No. CAS** | **Supplier** | **Purity** |
|---|---|---|---|
| TOA | 1116-76-3 | Merck | ≥ 93 % |
| TBP | 126-73-8 | Acros Organics | > 99 % |
| TOPO | 78-50-2 | Acros Organics | 99 % |
| n-tetradecane | 629-59-4 | Merck | > 99 % |
| 2-undecanone | 112-12-9 | Sigma Aldrich | 99 % |
| 1-undecanol | 112-42-5 | Sigma Aldrich | 99 % |
| Phenyldodecane | 123-01-03 | Alfa Aesar | 97 % |
| Sodium hydroxide solution | 1310-73-2 | Merck | 0.01 mol/L (0,1 N) |
| Acetonitrile | 75-05-8 | Carl Roth | > 99.9 % |
| Acetic acid | 64-19-7 | VWR Chemicals | 99.9 % |
| Di-n-butylether | 142-96-1 | Alfa Aesar | 99 % |
| Ethanol absolute | 64-17-5 | VWR Chemicals | 100 % |

**Table 2 shows some important characteristics of the tested extractants and diluents:**

| **Extractants** | **Boiling point [°C]** | **Molecular weight [g/mol]** | **Solubility in water** |
|---|---|---|---|
| TOA | 365-367^{[1]} | 353.68^{[1]} | < 5ppm^{[2]} |
| TBP | 293^{[3]} | 266.32^{[3]} | 6 g/L (20°C) ^{[4]} |
| TOPO | 213^{[5]} | 386.64^{[6]} | Insoluble^{[5]} |

| **Diluents** | | | |
|---|---|---|---|
| n-Tetradecane | 253^{[7]} | 198.39^{[7]} | 0.0022 mg/L^{[8]} |
| 2-Undecanone | 231^{[9]} | 170.29^{[9]} | Insoluble^{[9]} |
| 1 -Undecanol | 243^{[10]} | 172.31^{[10]} | Insoluble^{[10]} |
| Phenyldodecane | 331^{[11]} | 246.44^{[11]} | Insoluble^{[11]} |

| | | | |
|---|---|---|---|
| 1: http://www.sigmaaldrich.com/catalog/product/aldrich/t81000?lang=de&region=DE, 04.07.2016 2: Braun, T.; Ghersini, G. (1975): Extraction Chromatography, Elsevier Scientific Publishing Company, Amsterdam 3: http://gestis.itrust.de/nxt/gateway.dll/gestis_de/017680.xml?f=templates$fn=default.htm$3.0, 04.07.2016 4: https://roempp.thieme.de/roempp4.0/do/data/RD-20-02721, 04.07.2016 5: http://www.sciencelab.com/msds.php?msdsld=9925337/, 04.07.2016 6: http://www.chemspider.com/Chemical-Structure.59020.html?rid=076311d5-2678-47db-af29-41eac9e426bc&page_num=0, 04.07.2016 7: http://www.chemspider.com/Chemical-Structure.11883. html?rid=5339ae90-edb2-4a85-a2cb-69147c6e0572, 04.07.2016 8: Sutton, C.; Calder, J. A. (1974): Solubility of higher-molecular-weight normal-paraffins in distilled water and sea water. In: Environmental Science and Technology 8, 654-657 9: http://gestis.itrust.de/nxt/gateway.dll/gestis_de/491186.xml?f=templates$fn=default.htm$3.0, 04.07.2016 10: http://gestis.itrust.de/nxt/gateway.dll/gestis_de/491186.xml?f=templates$fn=default.htm$3.0, 04.07.2016 11: http://gestis.itrust.de/nxt/gateway.dll/gestis_de/491186.xml?f=templates$fn=default.htm$3.0,04.07.2016 | | | |

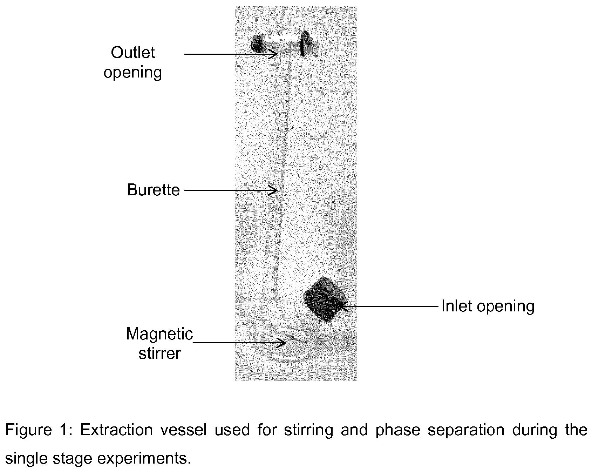

In a first step, a solvent screening was performed for each extractant/diluent combination given in Table 1, where the aqueous feed solution (consisting of water and acetic acid) contained 1.6 wt% HAc and the extractant concentration was varied between 15 and 50 wt%. Accordingly, the diluent concentration was varied between 85 and 50 wt%. Thereby, the distribution coefficient of HAc was chosen as performance indicator. In order to determine the distribution coefficient, single-stage extraction experiments were performed and both phases were analyzed by titration with phenolphthalein. For titration, a solution of 0.05 M NaOH was prepared, which was standardized using potassium biphthalate. The experiments were performed at ambient pressure, 20 °C and a mass-based phase ratio of 1. The samples were mixed for one hour and settled for at least 24 hours, while the temperature was kept constant by using a thermostatic bath. The mass of the phases after extraction was determined by weighing after separating the phases using a special extraction vessel shown in Figure 1. In order to avoid the formation of a second phase during the analysis of the organic phase, ethanol was added to the solution.

It is noted that the distribution coefficient is a good performance indicator as it describes the liquid-liquid equilibrium and it is therefore a measure for the ability of a solvent to concentrate a solute. It is also a commonly used performance indicator. The distribution coefficient was calculated by dividing the organic phase mass fraction by the aqueous phase mass fraction. The distribution coefficient is preferably at least 1.

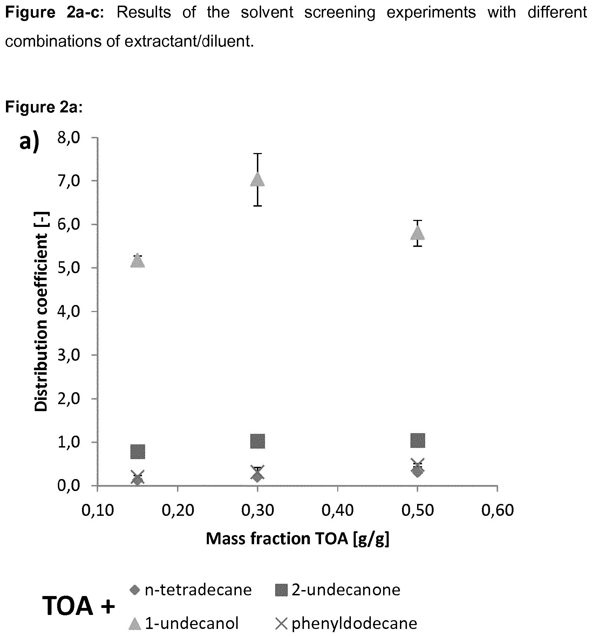

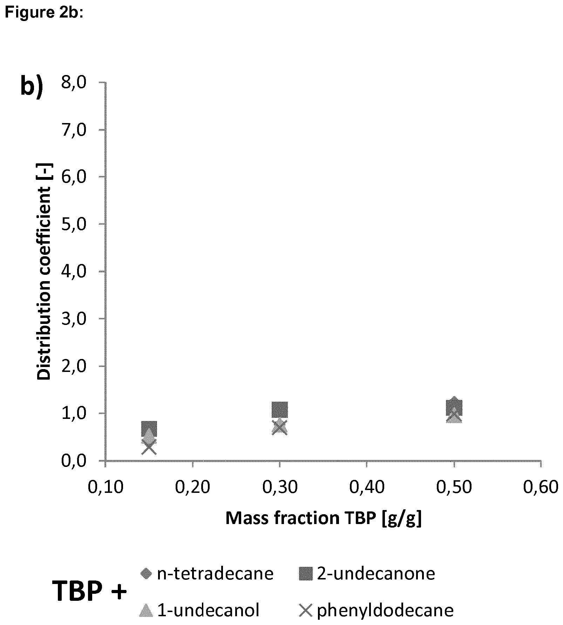

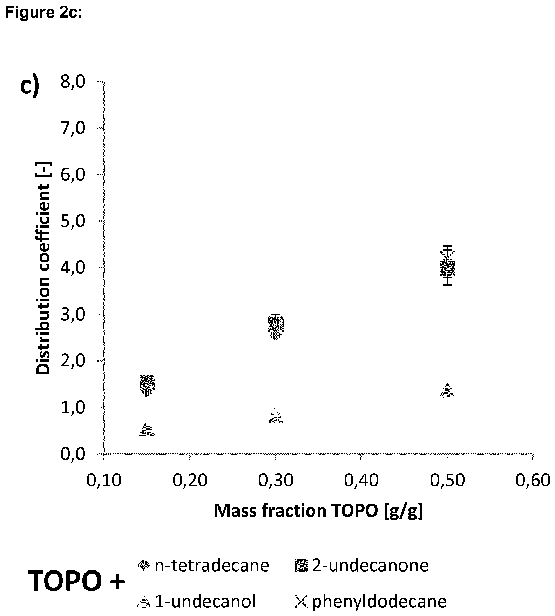

The results of this experiment are summarized in Figures 2a, 2b, and 2c. From these results it is clear that none of the combinations of TBP with the different diluents achieves beneficial distribution coefficients larger than one. The combinations TOPO/n-tetradecane, TOPO/2-undecanone and TOPO/phenyldodecane achieve good distribution coefficients for high extractant concentrations. Surprisingly, however, the combination TOA/1-undecanol leads to maximum distribution coefficients for all investigated extractant concentrations. The experiments in Figure 2a show that the performance of TOA combined with other solvents than 1-undecanol is much worse (distribution coefficients mostly below one), which implies that 1-undecanol is best suited for dissolving the complex of TOA and HAc compared to the other tested diluents.

In the first step of the more detailed investigation, the TOA content was further increased in order to test if the distribution coefficient can be further improved. For this experiment, the analytical method was changed to gas chromatography (GC) in order to be able to determine the concentrations of the other components besides HAc. The equipment was previously calibrated for the substances with different solutions of acetonitrile (ACN) and di-n-butylether (DBE) as internal standards for the aqueous and the organic phase, respectively. Each sample was introduced into a small vial with 0.4 g of the internal standard. Both phases were analyzed with GC equipment by Shimadzu, which has a flame ionization detector and uses helium as carrier gas. The specifications of the column were: FS-Supreme-5ms-HT from CS-Chromatographie-Service 30 m length, 0.32 mm inner diameter, with 0.25 µm film. A summary of the specifications of the method used by the GC is given in Table 2.

**Table 2. Summary of the GC methods used for analyzing the organic and aqueous phases.**

| **GC Shimadzu** | **Aqueous phase** | **Organic phase** |
|---|---|---|
| Injection temperature | 340 °C | 340 °C |
| Detector temperature | 360 °C | 360 °C |
| Method | Start temperature: 60°C hold for 2,3 min Heat up with 30°C/min to 100°C hold for 0,4 min | Start temperature: 60°C hold for 3,5 min Heat up with 30°C/min to 280°C Heat up with 15°C/min to 360°C hold for 0,1 min |
| Injection volume | 0.1 µL with 10 µL-syringe | 0.1 µL with 10 µL-syringe |

Similar to the first experiment, this experiment was performed at ambient pressure, 20 °C and a mass-based phase ratio of 1. The samples were mixed for one hour and settled for at least 24 hours, while the temperature was kept constant by using a thermostatic bath. The mass of the phases after extraction was again determined by weighing.

It was found that a ratio of TOA/1-undecanol above 0.8 leads to more favorable distribution coefficients of HAc. It was also found that the distribution coefficient decreases for an increasing HAc feed concentration.

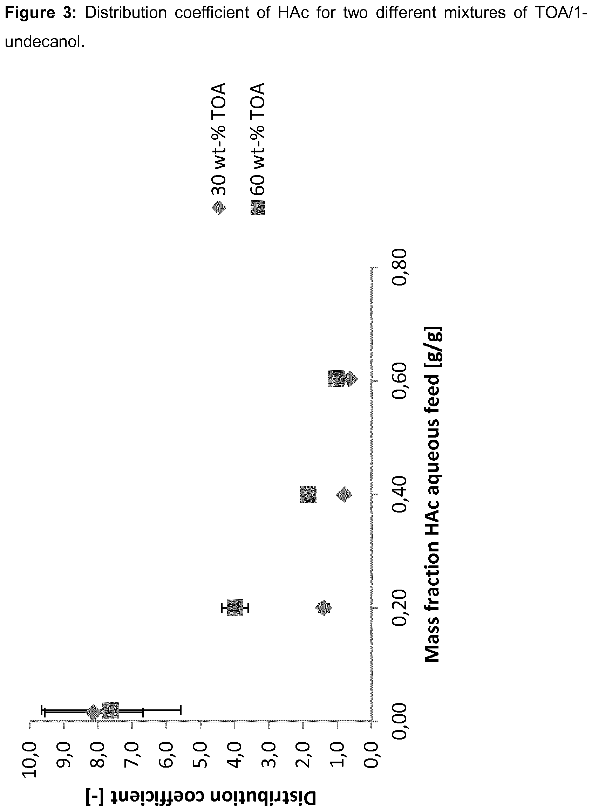

Figure 3 shows the results of the extraction of HAc from an aqueous solution with an increasing HAc feed concentration with a TOA/1-undecanol solvent mixture containing 60 wt% of TOA and 40 wt% of 1-undecanol; and 30 wt% of TOA and 70 wt% of 1-undecanol, respectively.
At 1.6 wt% HAc, both solvent mixtures achieve the same distribution coefficient of approx. 8, regardless of the TOA mass fraction. At 20 wt% HAc, however, the amount of TOA in the solvent mixture containing 30 wt% TOA is obviously not sufficient for effectively forming complexes with the HAc molecules, which leads to a distribution coefficient close to one. When the TOA concentration is increased to 60 wt%, the distribution coefficient at 20 wt% HAc is still around 4.

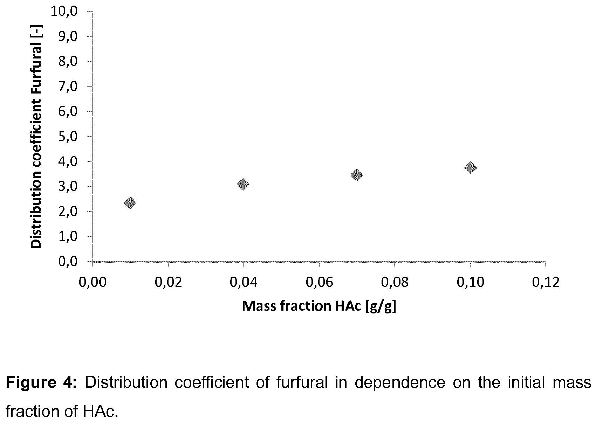

The distribution of furfural, a contamination which is sometimes present in the aqueous stream comprising acetic acid which is subjected to the process according to the present invention, was tested for a solvent: feed ratio of one, a solvent mixture containing 60 wt% TOA and 40 wt% 1-undecanol, a temperature of 20 °C as well as a constant initial mass fraction of furfural in the aqueous feed of 1 wt% and a range of mass fractions of HAc between 1 wt% and 10 wt%. The experiments were conducted according to the aforementioned procedure and both phases were analyzed by GC applying the methods summarized in Table 2. Figure 4 shows the distribution coefficient of furfural in dependence on the initial HAc mass fraction. The distribution coefficient remains almost constant around 3, with a slight increase towards higher HAc mass fractions.

## Claims

1. Process for recovering acetic acid from an aqueous stream comprising acetic acid, said process comprising the steps of
(a) contacting the aqueous stream with tri-n-octylamine and 1-undecanol, with the weight ratio between tri-n-octylamine and 1-undecanol being between 0.8 : 1 and 5 : 1, to produce
an organic phase (I) comprising acetic acid, tri-n-octylamine and 1-undecanol, and
an aqueous phase (II);
and
(b) isolating acetic acid from the organic phase (I).

2. The process according to claim 1 wherein the weight ratio between tri-n-octylamine and 1-undecanol is between 1 : 1 and 3 : 1, preferably between 1.5 : 1.

3. The process according to claim 1 or 2 wherein the aqueous stream comprises between 0.5 and 40 % by weight of acetic acid.

4. The process according any one of the preceding claims wherein the aqueous stream further comprises one or more compounds selected from the group consisting of methanol, furfural, formic acid, other organic acids, phenolics, furanics, sugar derivatives.

5. The process according to claim 4, wherein the aqueous stream originates from a process wherein bio-based chemicals are produced from lignocellulosic biomass.

6. The process of any one of the preceding claims wherein step (a) of the process is conducted at a temperature of 15 to 60 °C, preferably between 20 and 45 °C, and a pressure of between 0.1 and 1 MPa, and preferably atmospheric pressure.

7. The process according to any one of the preceding claims wherein in step (b) acetic acid is isolated from the organic phase via one or more distillation steps.

## Patentansprüche

1. Verfahren zur Gewinnung von Essigsäure aus einem wässrigen Strom umfassend Essigsäure, wobei das Verfahren die Schritte umfasst des
(a) Kontaktierens des wässrigen Stroms mit Tri-n-octylamin und 1-Undecanol, wobei das Gewichtsverhältnis zwischen Tri-n-octylamin und 1-Undecanol zwischen 0,8 : 1 und 5 : 1 liegt, um
eine organische Phase (I), die Essigsäure, Tri-n-octylamin und 1-Undecanol umfasst, und
eine wässrige Phase (II) herzustellen
und
(b) Isolierens von Essigsäure von der organischen Phase (I).

2. Verfahren nach Anspruch 1, wobei das Gewichtsverhältnis zwischen Tri-n-octylamin und 1-Undecanol zwischen 1 : 1 und 3 : 1, bevorzugt zwischen 1,5 : 1 liegt.

3. Verfahren nach Anspruch 1 oder 2, wobei der wässrige Strom zwischen 0,5 und 40 Gew.-% Essigsäure umfasst.

4. Verfahren nach einem der vorhergehenden Ansprüche, wobei der wässrige Strom ferner eine oder mehrere Verbindungen umfasst ausgewählt aus der Gruppe bestehend aus Methanol, Furfural, Ameisensäure, anderen organischen Säuren, phenolischen Substanzen, furanischen Substanzen, Zuckerderivaten.

5. Verfahren nach Anspruch 4, wobei der wässrige Strom aus einem Verfahren stammt, wobei biobasierte Chemikalien aus lignocellulosischer Biomasse hergestellt werden.

6. Verfahren nach einem der vorhergehenden Ansprüche, wobei der Schritt (a) des Verfahrens bei einer Temperatur von 15 bis 60 °C, bevorzugt zwischen 20 und 45 °C und bei einem Druck zwischen 0,1 und 1 MPa und bevorzugt bei Luftdruck ausgeführt wird.

7. Verfahren nach einem der vorhergehenden Ansprüche, wobei in Schritt (b) Essigsäure von der organischen Phase durch einen oder mehrere Destillationsschritte isoliert wird.

## Revendications

1. Procédé de récupération d'acide acétique à partir d'un courant aqueux comprenant de l'acide acétique, ledit procédé comprenant les étapes de
(a) mise en contact du courant aqueux avec de la tri-n-octylamine et du 1-undécanol, le rapport pondéral entre la tri-n-octylamine et le 1-undécanol étant compris entre 0,8:1 et 5:1, pour produire
une phase organique (I) comprenant de l'acide acétique, de la tri-n-octylamine et du 1-undécanol, et
une phase aqueuse (II) ;
et
(b) isolement de l'acide acétique de la phase organique (I).

2. Procédé selon la revendication 1 dans lequel le rapport pondéral entre la tri-n-octylamine et le 1-undécanol est compris entre 1:1 et 3:1, de préférence entre 1,5:1.

3. Procédé selon la revendication 1 ou 2 dans lequel le courant aqueux comprend entre 0,5 et 40 % en poids d'acide acétique.

4. Procédé selon l'une quelconque des revendications précédentes dans lequel le courant aqueux comprend en outre un ou plusieurs composés sélectionnés dans le groupe constitué par le méthanol, le furfural, l'acide formique, d'autres acides organiques, les composés phénoliques, les composés furaniques, les dérivés de sucre.

5. Procédé selon la revendication 4, dans lequel le courant aqueux provient d'un procédé dans lequel des produits chimiques d'origine biologique sont produits à partir de biomasse lignocellulosique.

6. Procédé selon l'une quelconque des revendications précédentes dans lequel l'étape (a) du procédé est réalisée à une température de 15 à 60 °C, de préférence entre 20 et 45 °C, et à une pression comprise entre 0,1 et 1 MPa, et de préférence à la pression atmosphérique.

7. Procédé selon l'une quelconque des revendications précédentes dans lequel dans l'étape (b), l'acide acétique est isolé de la phase organique par le biais d'une ou plusieurs étapes de distillation.
